# EUROPEAN PATENT SPECIFICATION

(11) **EP 4 410 185 B1**
(45) Date of publication and mention of the grant of the patent: **10.09.2025**
(21) Application number: 23220720.9
(22) Date of filing: 29.12.2023
(51) Int. Cl.: A61B 5/00, A61B 5/287

(54) **ARRHYTHMIA DIAGNOSIS ELECTRODE APPARATUS HAVING REDUCED DIRECTIONAL STIFFNESS**
ARRHYTHMIE-DIAGNOSEELEKTRODENVORRICHTUNG MIT REDUZIERTER DIREKTIONALER STEIFIGKEIT
APPAREIL À ÉLECTRODE DE DIAGNOSTIC D'ARYTHMIE AYANT UNE RIGIDITÉ DIRECTIONNELLE RÉDUITE

(30) Priority: 30.12.2022 US 202263477930 P; 09.11.2023 US 202318505789
(43) Date of publication of application: 07.08.2024
(73) Proprietor: BIOSENSE WEBSTER (ISRAEL) LTD., 2066717 Yokneam (IL)
(72) Inventor: VAN NIEKERK, Pieter Emmelius, Irvine, 92618 (US); PATEL, Amar, Irvine, 92618 (US); TOBEY, Dustin R., Irvine, 92618 (US)
(74) Representative: Carpmaels & Ransford LLP

(56) References cited:
- EP-A1- 3 915 477
- US-A1- 2017 056 105
- US-A1- 2021 369 339

## Description

### FIELD

The present invention relates generally to minimally invasive medical devices, and in particular sensing catheters, and further relates to, but not exclusively, cardiac mapping catheters.

### BACKGROUND

EP 3915477 A1 discusses an apparatus including an end effector having loop members with electrodes thereon and which is usable with catheter-based systems to measure or provide electrical signals. The end effector can include three loop members that are non-coplanar when expanded unconstrained that become contiguous to a planar surface when the loop members are deflected against the surface, a mechanical linkage that joins the loop members at a distal vertex of the end effector, electrodes having surface treatment to enhance surface roughness of the electrodes, twisted pair electrode wires, a bonded spine cover, and/or any combination thereof.

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature.

### SUMMARY

There is provided, in accordance with an embodiment of the present invention, a loop assembly for a catheter that can include a primary frame member, a secondary frame member, and a sleeve disposed around least one of the primary and secondary frame members. The primary frame member can have at least a first cross-sectional area and include a pair of proximal ends extending along a longitudinal axis to a closed distal end. The secondary frame member can have at least a second cross-sectional area different from the first cross-sectional area, and be disposed on at least a portion of the primary frame member between the proximal ends and the closed distal end. The secondary frame member can be configured to slide in at least a first dimension (e.g., longitudinally) relative to the primary frame member.

The disclosed technology includes an end effector for use with a catheter shaft. The end effector can include a plurality of loop members, with each loop member having a support frame including a primary frame member and a secondary frame member. The primary frame member can have at least a first cross-sectional area and a secondary frame member can have at least a second cross-sectional area different from the first cross-sectional area. The secondary frame member can be disposed on a portion of the primary frame member and constrained to slide along a portion of the first frame member. The primary frame member can define a pair of proximal ends and a closed distal end spaced apart along a longitudinal axis.

The disclosed technology includes an assembly for a mapping catheter that can include a tubular member extending along a longitudinal axis, a plurality of loop members each having a support frame affixed to a distal portion of the tubular member, and a plurality of electrodes disposed on the respective support frame. The support frame can extend through the respective loop member to define two spine members extending from the tubular member and connected to an arcuate member. At least a portion of each of the spine members of at least a first support frame can have longitudinally extending first and second layers. The first layer of the first support frame can be configured to slide in at least a first dimension relative to the second layer of the first support frame.

The disclosed technology includes a method that can include shaping at least a first loop member and a second loop member to each form a respective loop, coupling a respective pair of ends for each of the at least first and second loop members to a distal portion of an elongated shaft extending along a longitudinal axis, and overlapping the at least first and second loop members at a common distal vertex distal to the distal portion of the elongated shaft such that when the at least first and second loop members are unconstrained, a majority of the first loop member being non-coplanar with a majority of the second loop member.

The method can also include pressing the majority of the first loop member and the majority of the second loop member in contact with a planar surface via manipulation of the elongated shaft, and contacting the at least first and second loop members with the planar surface to align the majorities of the at least first and second loop members with the planar surface away from the longitudinal axis, thereby causing the first layer of the spines of at least the first loop member to longitudinally slide relative to the second layer of the respective spines.

Each of the first and second loop members can include a support frame including two spines, with the spines of at least the first loop member each including a first layer extending longitudinally along a first length of the respective spine and a second layer extending longitudinally along a second length of the respective spine overlapping the first length, and first layer being configured to longitudinally slide relative to the second layer of each respective spine.

### BRIEF DESCRIPTION OF THE DRAWINGS

FIG. 1A is an illustration of a catheter having an end effector at a distal portion of the catheter and a deflection control handle at a proximal portion of the catheter, in accordance with an embodiment of the present invention;
FIG. 1B is an illustration of an orthogonal side plan view of a catheter, in accordance with an embodiment of the present invention;
FIG. 1C is an illustration of an orthogonal side plan view of a catheter, in accordance with an embodiment of the present invention;
FIG. 2A is an illustration of a top view of the end effector, in accordance with an embodiment of the present invention;
FIG. 2B is an illustration of a side view of the end effector, in accordance with an embodiment of the present invention;
FIG. 3A is an illustration of a rear view of the end effector pressed to a planar surface, in accordance with an embodiment of the present invention;
FIG. 3B is an illustration of a side view of the end effector pressed to a planar surface, in accordance with an embodiment of the present invention;
FIG. 4 is an illustration of a view of support frames of the end effector in an unconstrained configuration, in accordance with an embodiment of the present invention;
FIG. 5A is an illustration of a support frame of the end effector, in accordance with an embodiment of the present invention;
FIG. 5B is an illustration of a cross sectional view of the support frame as indicated by B-B in FIG. 5A;
FIG. 5C is an illustration of a cross sectional view of the support frame as indicated by A-A in FIG. 5A;
FIG. 6A is an illustration of a loop assembly for a catheter, in accordance with an embodiment of the present invention;
FIG. 6B is an illustration of a cross sectional view of a loop assembly for a catheter as indicated by A-A in FIG. 6A;
FIG. 6C is an illustration of cross sectional view a loop assembly for a catheter as indicated by B-B in FIG. 6A;
FIG. 6D is an illustration of a detailed section of a loop assembly for a catheter as indicated by D-D in FIG. 6A;
FIG. 6E is an illustration of a cross sectional view of a loop assembly for a catheter having a primary frame member and a secondary frame member as indicated by E-E in FIG. 6A;
FIG. 6F is an illustration of a cross sectional view of a loop assembly for a catheter having a primary frame member and a secondary frame member as indicated by B-B in FIG. 6A;
FIG. 6G is an illustration of a side cross section view of a loop assembly for a catheter having a primary frame member and a secondary frame member as indicated by B-B in FIG. 6A;
FIG. 6H is an illustration of a side cross section view of the loop assembly of FIG. 6A being bent;
FIG. 7A is an illustration of deformation of the support frames as a result of application of various forces, in accordance with an embodiment of the present invention;
FIG. 7B is an illustration of deformation of the support frames as a result of application of various forces, in accordance with an embodiment of the present invention;
FIG. 7C is an illustration of deformation of the support frames as a result of application of various forces, in accordance with an embodiment of the present invention;
FIG. 7D is an illustration of deformation of the support frames as a result of application of various forces, in accordance with an embodiment of the present invention;
FIG. 8 is a flowchart illustrating a method of making an end effector, in accordance with an embodiment of the present invention;
FIG. 9 is a flowchart illustrating a method of making an end effector, in accordance with an embodiment of the present invention;
FIG. 10 is a flowchart illustrating a method of making an end effector, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, or any other individual or delivery instrumentation associated with delivery of a multi-electrode catheter for cardiac mapping of a subject.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

In order to enhance the ability of the catheter disclosed herein to be adaptable to the flat, curved, irregular and/or nonplanar tissue surfaces found in the target anatomy, the catheter has reduced directional stiffness, namely, but not exclusively, in the longitudinal direction. This is achieved in part by allowing constituent components of a loop assembly of an end effector of the catheter to slide relative to one another in the longitudinal direction i.e., along their respective lengths.

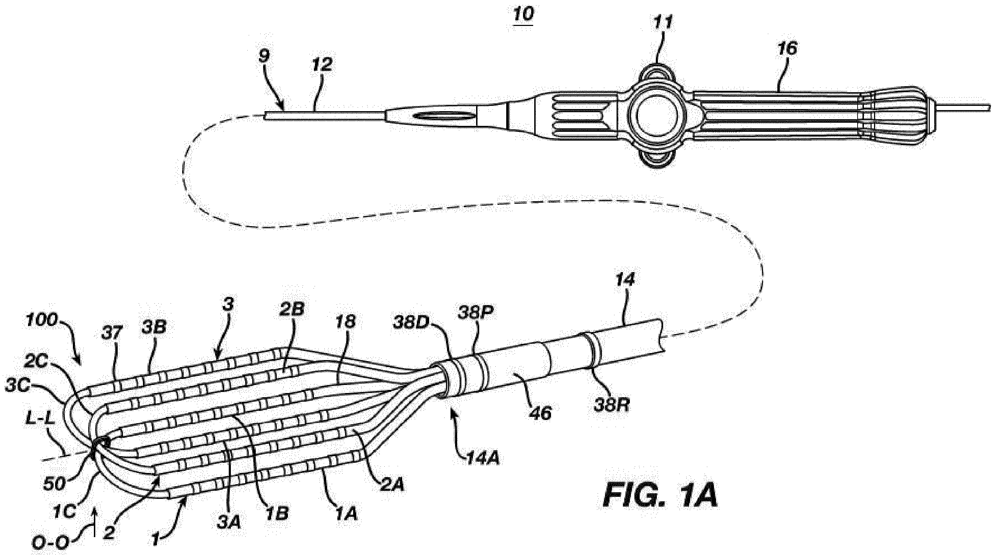

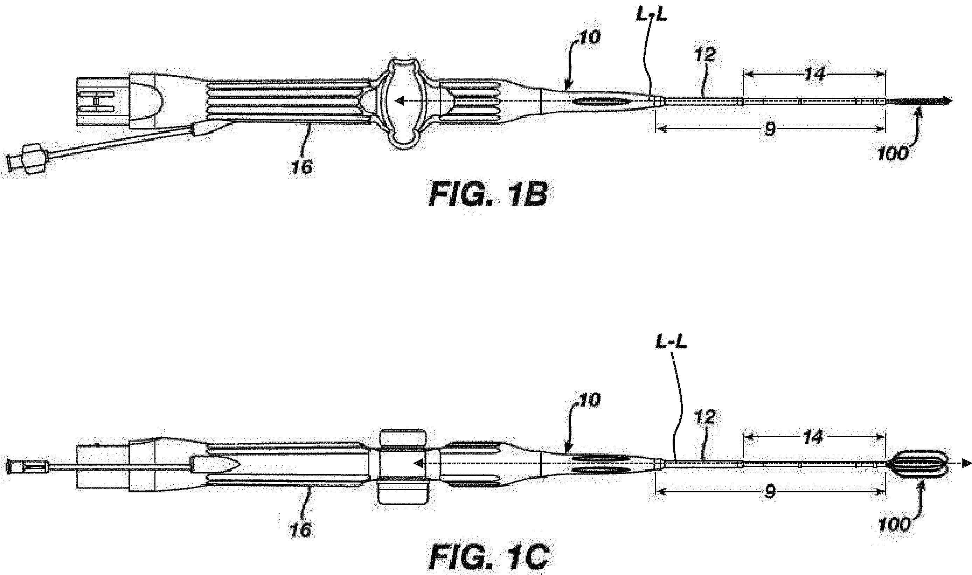

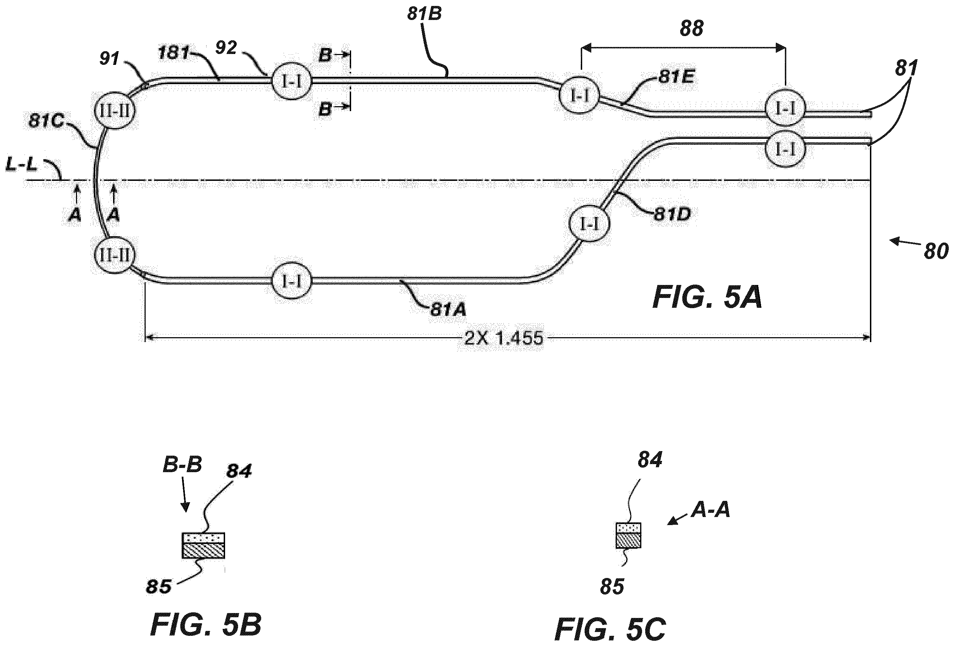

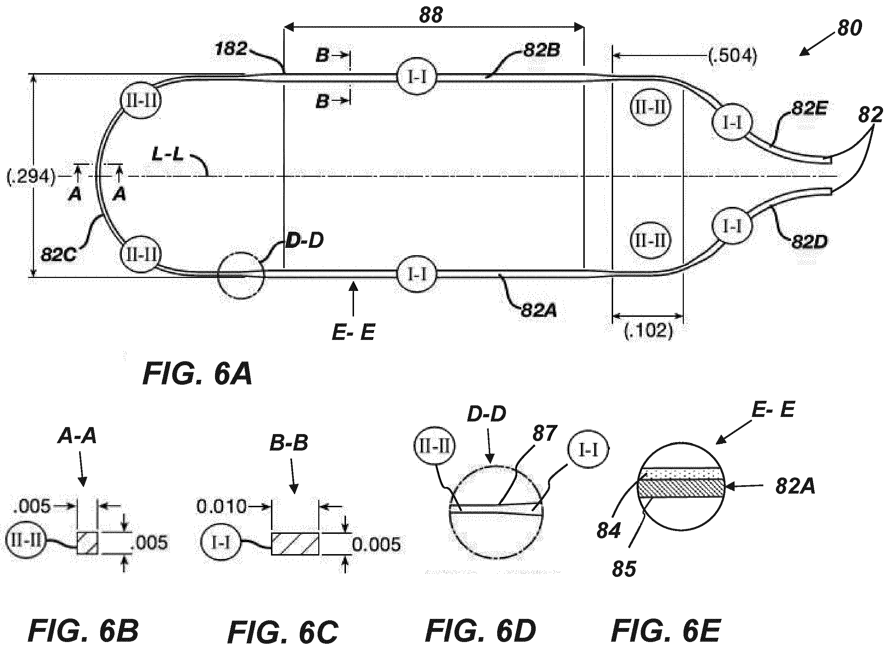

Reference is made to FIG. 1A showing an assembly for a mapping catheter. FIG. 1B and FIG. 1C show side views of the assembly. The assembly can include a tubular member 9 extending along a longitudinal axis L-L. The assembly can also include a plurality of loop members 1, 2, 3 each having a support frame 1A-E, 2A-E, 3A-E affixed to a distal portion 14A of the tubular member 9. The support frames 1A-E, 2A-E, 3A-E can extend through the respective loop member 1, 2, 3 to define two spine members 1A and 1B, 2A and 2B, 3A and 3B extending from the tubular member 9 and connected to an arcuate member 1C, 2C, 3C. A plurality of electrodes 37 can be disposed on the respective support frame 1, 2, 3. Though not readily visible in FIGs. 1A-1C, cross-section views of FIGs. 6A-G. show at least a portion of each of the spine members 1A and 1B of at least a first support frame 1A-E of the support frames 1A-E, 2A-E, 3A-E show that at least a portion of each of the spine members can include longitudinally extending first 84 and second 85 layers, the first layer 84 of the first support frame 1 being configured to slide in at least a first dimension relative to the second layer 85 of the first support frame 1. Specific aspects and dimensions relating to the first 84 and second 85 layers will be described in relation to FIGs. 5A-6G.

In some examples, the first dimension is longitudinal. In some examples, a first loop member 1 of the plurality of loop members 1, 2, 3 is arrayed on a first plane S1, a second loop member 2 of the plurality of loop members 1, 2, 3 is arrayed on a second plane S2 that intersects with the first plane S1, and a third loop member 3 of the plurality of loop members 1, 2, 3 is arrayed on a third plane that intersects with the first plane S1 and the second plane S2 such that a majority of a length of each of the three loop members 1, 2, 3 is non-coplanar with the majority of the length of at least one of the other three loop members in an unconstrained configuration.

In some examples, the plurality of loop members 1, 2, 3 each includes an inner tubular housing 91 surrounding at least a portion of the respective support frame and an outer tubular housing 92 surrounding at least a portion of the inner tubular housing 91 and bonded to the inner tubular housing 91. The plurality of electrodes 37 can be disposed at least partially within the outer tubular housing 92 and outside of the inner tubular housing 91 of the respective loop members.

The assembly 10 can also include an end effector 100 at a distal portion 14A of the catheter 10 and a deflection control handle 16 at a proximal portion 12 of the catheter 10.

As shown in the example shown in FIG. 1A, the catheter 10 can include an elongated shaft 9. The shaft 9 is preferably a tubular member. The elongated shaft 9 has a proximal portion 12 in the shape of an elongated catheter body, an intermediate deflection section 14, and distal portion 14A. The deflection control handle 16 is attached to the proximal end of the catheter body 12. Suitable control handles are disclosed in US Patent Nos. 6,123,699; 6,171,277; 6,183,435; 6,183,463; 6,198,974; 6,210,407 and 6,267,746, each of which is included in the appendix included with priority application no. 63/477,930. The distal portion 14A of the shaft is coupled to the end effector 100 via a connector tubing 46. The elongated shaft 9 forms a tubular catheter body sized and otherwise configured to traverse vasculature. The end effector 100 has a plurality of loop members 1, 2, 3 that overlap at a common distal vertex and are joined at the distal vertex 50.

The proximal portion 12 can be a catheter body including an elongated tubular construction having a single axial passage or central lumen 18. The catheter body 12 is flexible, i.e., bendable, but substantially non-compressible along its length. The catheter body 12 can be of any suitable construction and made of any suitable material. In some embodiments, the catheter body 12 has an outer wall made of polyurethane or PEBAX. The outer wall may include an imbedded braided mesh of stainless steel or the like to increase torsional stiffness of the catheter body 12 so that, when the control handle 16 is rotated, the intermediate section 14 will rotate in a corresponding manner.

The outer diameter of the catheter body 12 is preferably no more than about 8 French, more preferably about 7 French. The thickness of the outer wall is thin enough so that the central lumen 18 can accommodate at least one puller wire, one or more lead wires, and any other desired wires, cables or tubes. If desired, the inner surface of the outer wall is lined with a stiffening tube 22 to provide improved torsional stability. In some embodiments, the outer wall has an outer diameter of from about 0.090 inch to about 0.094 inch (from about 2.3 mm to about 2.4 mm) and an inner diameter of from about 0.061 inch to about 0.065 inch (from about 1.5 mm to about 1.7 mm).

The distal portion 14A of the shaft 9 is coupled to the end effector 100 with a connector tubing 46. The connector tubing 46 includes an insert for connection of loop members 1, 2, 3 to provide electrical connection through the intermediate portion 14 of the catheter body. The connector tubing 46 can be affixed to the distal portion 14A of the catheter by glue or the like.

The distal portion 14A of the shaft 9 can be substantially contiguous with the intermediate section 14 such that the intermediate section comprises the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14.

When the device is unconstrained and aligned, the proximal portion 12, intermediate section 14, distal portion 14A, and end effector 100 are generally aligned along a longitudinal axis L-L. The intermediate section 14 can be configured to bend to deflect the distal portion 14A and end effector 100 from the longitudinal axis L-L.

The end effector 100 can be collapsed (compressed toward the longitudinal axis L-L) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The connectors 1C, 2C, 3C are preferably arcuate members as illustrated.

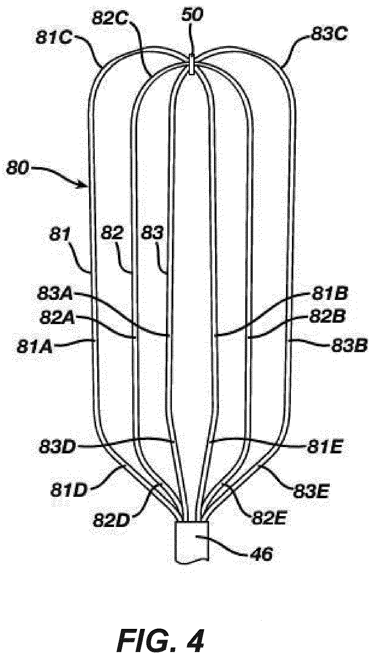

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in FIG. 1A. Preferably, all spines in the end effector are parallel to each other along the majority of their respective lengths when the end effector 100 is in the unconstrained configuration. Even when all spines are parallel, the spines are not necessarily all coplanar as described in greater detail elsewhere herein, for instance in relation to FIG. 4.

Each spine 1A, 1B, 2A, 2B, 3A or 3B can have a length ranging between about 5 and 50 mm, preferably about 10 and 35 mm, and more preferably about 28 mm. The parallel portions of each spine 1A, 1B, 2A, 2B, 3A, 3B can be spaced apart from each other by a distance ranging between about 1mm and 20 mm, preferably about 2 and 10 mm, and more preferably about 4 mm. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes.

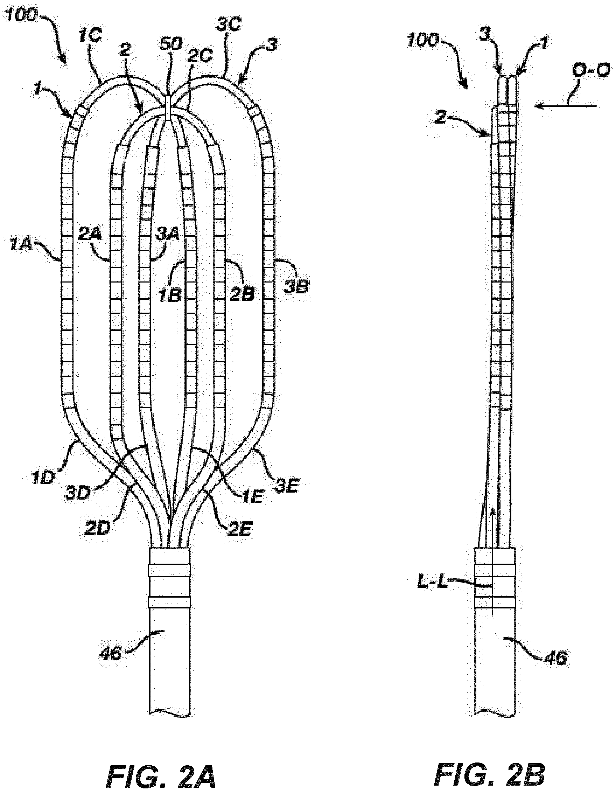

Reference is made to FIG. 2A and FIG. 2B showing illustrations of a top and side view of the end effector 100. The three loop members 1, 2, 3 overlap at a common distal vertex 50 along the longitudinal axis L-L. Each of the loop members 1, 2, 3, respectively include proximal end segments 1D, 2D, 3D, 1E, 2E, 3E affixed to the distal portion 14A of the elongated shaft 9 of the apparatus 10.

The end effector 100 is in an unconstrained configuration as illustrated in FIGs. 2A and 2B. As better visualized in the side view of FIG. 2B, when the end effector is unconstrained, the loop members 1, 2, 3 are not coplanar with each other. FIG. 2B also illustrates an orthogonal axis O-O orthogonal to the longitudinal axis L-L and approximately orthogonal to the top view of the end effector 100.

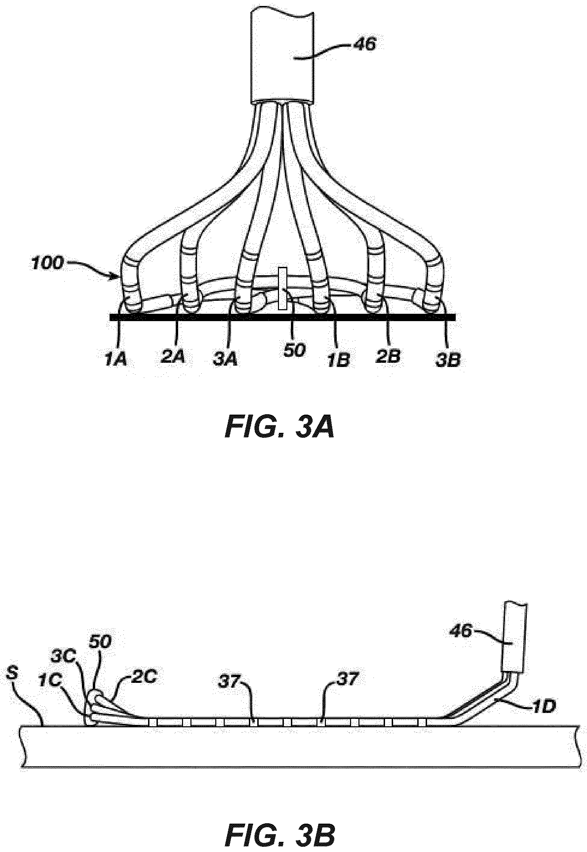

FIGs. 3A and 3B are illustrations of views of the end effector pressed to a planar surface S. In the illustrated example, the loop members 1, 2, 3 can be pressed to the planar surface S via manipulation of the shaft 9 of the device. More specifically, when the end effector 100 is positioned within a patient, manipulation of the catheter body 12 and the control handle 16 can be used to position the end effector 100 against a surface within a wall of an internal cavity of the patient such internal walls of the heart and/or blood vessels. When the end effector 100 is positioned against the planar surface S, a majority of each length of each spine 1A, 2A, 3A, 1B, 2B, 3B can become contiguous and aligned to the planar surface. Further, when the end effector 100 is positioned against the planar surface S, a majority of each length of each spine 1A, 2A, 3A, 1B, 2B, 3B can become aligned with a majority of each length of the other spines. The surface S need not necessarily be planar in order for the spines 1A, 2A, 3A, 1B, 2B, 3B to become contiguous and aligned to the surface. The end effector 100 may be able to conform to a curved surface, for instance.

As illustrated in FIG. 3B, when the majority of each spine 1A, 2A, 3A, 1B, 2B, 3B is pressed to the surface S, the connecting segments 1C, 2C, 3C can be stacked on top of the surface S at the distal vertex at the linkage 50. A first connecting segment 1C nearest to the surface S can be separated from the surface S by the linkage 50. A second connecting segment 3C stacked onto the first connecting segment 1C can be separated from the surface S by the linkage 50 and the first connecting segment 1C. A third connecting segment 2C can be separated from the surface S by the linkage 50 and the first and second connecting segments 1C, 3C. Therefore, at least a portion of each connecting segment 1C, 2C, 3C can be separated from the planar surface S when the majority of each spine 1A, 2A, 3A, 1B, 2B, 3B is pressed to the planar surface. Alternatively, the linkage 50 can be inset into the first connecting segment 1C such that the first connecting segment is substantially contiguous to the planar surface S. In that case, only the second and third connecting segments 3C, 2C are separated from the planar surface S at the distal vertex.

Proximal segments 1D, 2D, 3D, 1E, 2E, 3E of the loop members 1, 2, 3 can be bent such that at least a portion of each of the proximal segments curves away from the surface S.

When the majority of each spine 1A, 2A, 3A, 1B, 2B, 3B is pressed to the surface S, at least some of the electrodes 37 on each spine can be in contact with the surface S. In some examples, every electrode 37 on each spine can be in contact with the surface 37.

When the majority of each spine 1A, 2A, 3A, 1B, 2B, 3B is pressed to the surface S, the majority of each respective length of each loop member can become contiguous to the surface S, where the respective length of each loop member includes the length of the respective loop member's spines 1A, 2A, 3A, 1B, 2B, 3B, connectors 1C, 2C, 3C, and proximal segments 1D, 2D, 3D, 1E, 2E, 3E (distal to the connector tubing 46).

FIG. 4 is an illustration of a loop assembly 80 of the end effector 100. When the end effector 100 is assembled, the loop members 1, 2, 3 each includes a respective support frame 81, 82, 83. The loop assembly 80 extends into the connector tubing 46 to mechanically affix the loop members 1, 2, 3 to the shaft 9. The support frames 81, 82, 83 provide structural integrity for the loop members 1, 2, 3. The support frames 81, 82, 83 can include plastic or metal cut-off sheets, plastic or metal round wire, plastic or metal square wire, or other suitable biocompatible material. In the preferred embodiments, the support frame are made from shape memory material such as, for example, nitinol. For the purposes of testing and illustration, the loop assembly 80 is joined at a distal vertex with mechanical linkage 50. In the assembled end effector 100, the support frames 81, 82, 83 can be assembled by virtue of a mechanical linkage affixed to an outer housing of the loop members 1, 2, 3 or a direct linkage between the support frames 81, 82, 83.

When the end effector is unconstrained, each of the respective support frames 81, 82, 83 defines a respective looped path of its respective loop member 1, 2, 3 as illustrated in FIG. 4. Each support frame 81, 82, 83 includes respective parallel segments 81A, 82A, 83A, 81B, 82B, 83B that extend through corresponding spines 1A, 2A, 3A, 1B, 2B, 3B of the end effector 100. Each support frame 81, 82, 83 includes respective proximal segments 81D, 82D, 83D, 81E, 82E, 83E that extend through corresponding proximal segments 1D, 2D, 3D, 1E, 2E, 3E of respective loop members 1, 2, 3. The proximal segments 81D, 82D, 83D, 81E, 82E, 83E extend into the connector tubing 46 to join the end effector 100 to the shaft 9. Each support frame 81, 82, 83 includes a respective connecting segments 81C, 82C, 83C that extends between the respective pair of parallel segments 81A, 82A, 83A, 81B, 82B, 83B and through the respective connecting segment 1C, 2C, 3C of the respective loop member 1, 2, 3.

Each support frame 81, 82, 83 can include knuckles 87 to promote conformance of the loop members 1, 2, 3 to the surface S, for example as shown in FIG. 6D The knuckles 87 can be spaced uniformly or non-uniformly along the looped path of a respective support member 81, 82, 83. Knuckle features can include thinned out sections of the material of the support members 81, 82, 83. Additionally, or alternatively, the knuckle features can include hinge mechanisms.

Each of the support frames 80 can respectively have a cross sectional shape that varies along the individual support frame's looped path, where the cross-sectional shape is taken from a cross section orthogonal to the direction of the looped path. Example support frame assemblies can have regions with a cross sectional area configured to resist deflection and/or other regions configured to facilitate deflection. The regions configured to resist deflection can have a larger cross-sectional area compared to the regions configured to facilitate deflection. Alternatively, the regions configured to resist deflection can be flattened to resist deflection in the direction of long sides of the cross section while having a similar cross section to non-flattened, or less flattened regions. That is, the thin section are intended to promote sheath retraction (lower force for the frame to collapse) while the distal section is intended still for frame collapsing, but the proximal section is intended to promote deflection with respect to the longitudinal axis L-L.

A support frame 81, 82, 83 including rectangular cross sections can be formed by selecting a sheet and cutting the sheet to the shape of each respective support frame 81, 82, 83, varying the width of each segment of each support frame 81, 82, 83 to be wider in regions. Alternatively, the support frame 81, 82, 83 can be formed by selecting square or rectangular wire, shaping the wire to form a looped path, and flattening the wire to have regions with wider cross sections and narrower cross sections.

A support frame 81, 82, 83 including ovoid or oval-shaped cross sections can be formed by selecting a round or oval wire, shaping the wire to form a looped path, and flattening the wire to have regions with wider cross sections and narrower cross sections.

FIG. 5A is an illustration of an asymmetrical support frame 181 of the end effector 100. FIGs. 5B and 5C are illustrations of cross-sectional views, as indicated by B-B and A-A respectively, of the asymmetrical support frame 181 as indicated in FIG. 5A. The asymmetrical support frame 181 is another example support frame that can be used in place of the outer support frames 81, 83. The symmetrical support frame 182 shown in FIG. 6A is another example support frame that can be used in place of the central support frame 82. In some examples, the loop assembly 80 can include two asymmetrical support frames 181 and a single symmetrical support frame 182.

FIG. 5A illustrates that the parallel segments 81A, 81B of the asymmetric support frame 181 can have a wider cross section I-I as illustrated in FIG. 5C compared to the narrower cross section II-II of the connecting segment 81C as illustrated in FIG. 5B. In some examples, the cross-sectional shape of the parallel segments 81A, 81B can be substantially rectangular with a width in the plane of the support frame 181 of about 0.013 inches (0.33 millimeters) and a height orthogonal to the plane of the support frame 181 of about 0.005 inches (0.13 millimeters). In some examples, the cross-sectional shape of the connecting segment 81C can be substantially rectangular or square with a width in the plane of the support frame 181 of about 0.008 inches (0.2 millimeters) and a height orthogonal to the plane of the support frame 181 of about 0.008 inches (0.2 millimeters). Further, the proximal segments 81E, 81D can have a cross section I-I of approximately the same dimensions as the parallel segments 81A, 81B.

As shown in FIGs. 5B and 5C, the asymmetric support frame 181 can include a primary frame member 84 having at least a first cross-sectional area II-II and a secondary frame member 85 having at least a second cross-sectional area I-I different from the first cross-sectional area II-II. The primary frame member 84 can include a pair of proximal ends 81D, 81E extending along a longitudinal axis L-L to a closed distal end 81C. The secondary frame member 85 can be disposed on at least a portion of the primary frame member 84 between the proximal ends 81D, 81E and the closed distal end 81C and can be configured to slide in at least a first dimension relative to the primary frame member 84. As will be described in greater detail herein in relation to FIGs. 6A-6G, because of the primary frame member 84 and the secondary frame member 85, the end effector 100 can have properties and features not previously incorporated into similar end effectors. Thus, it will be appreciated that the features of the primary frame member 84 and the secondary frame member 85 described further herein in relation to FIGs. 6A-6G can be applicable to the primary frame member 84 and the secondary frame member 85 shown in FIGs. 5A-5C. Furthermore, although not shown, it will be appreciated that a cross-section of the end effector can have only a primary frame member 84 and only a secondary frame member 85 at given locations along the end effector 100.

Reference is made to FIGs. 6A-6G showing a loop assembly 80 for a catheter. In some examples, the assembly can include a primary frame member 84 having at least a first cross-sectional area II-II and a secondary frame member 85 having at least a second cross-sectional area I-I different from the first cross-sectional area II-II. The primary frame member 84 can include a pair of proximal ends 82D, 82E extending along a longitudinal axis L-L to a closed distal end 82C. The secondary frame member 85 can be disposed on at least a portion of the primary frame member 84 between the proximal ends 82D, 82E and the closed distal end 82C and can be configured to slide in at least a first dimension relative to the primary frame member 84.

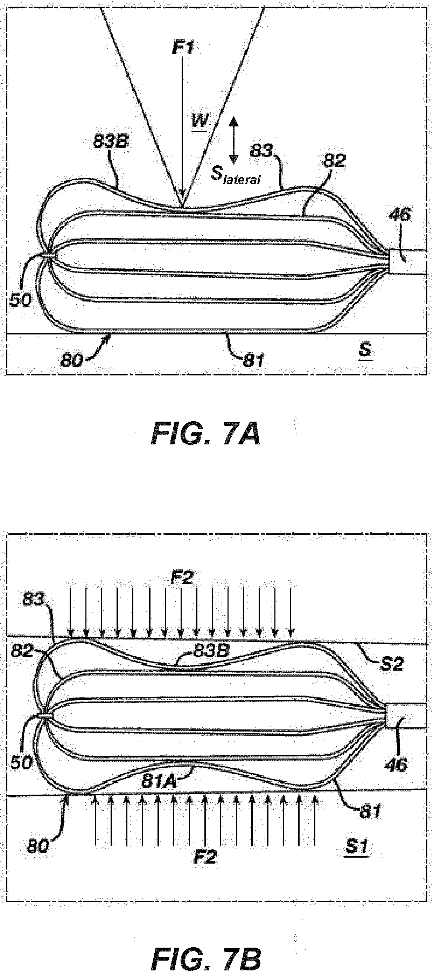

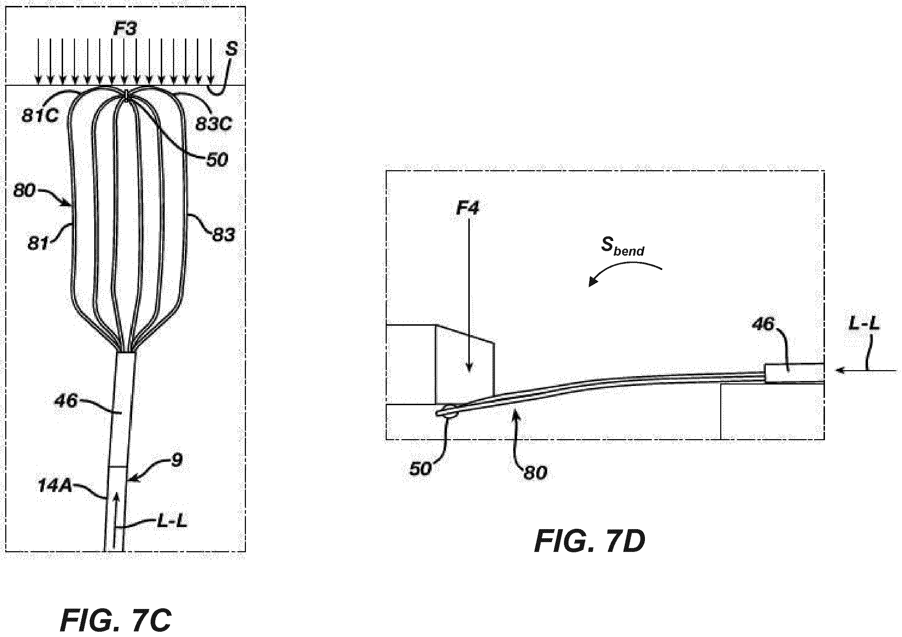

In some examples, the first dimension L-L is longitudinal. In some examples, the primary frame member 84 can be a metallic or polymeric strip and longitudinally extending along at least a first portion 82A of a length of the loop assembly 80, and the secondary frame member 85 can be a metallic base and longitudinally extending along at least a second portion 82B of the length 82 of the loop assembly 80. The first portion 82A of the length 82 of the loop assembly 80 can at least partially overlap the second portion 82B of the length 82 of the loop assembly 80 at an overlapping section 88. In some examples, such as that shown in FIG. 6A the overlapping section 88 can extend along the entirety of portions 82A and 82B. This configuration limits the increase in bending stiffness S_{bend} (for example as shown in FIG. 7D) while appropriately increasing lateral stiffness Sₗₐₜₑᵣₐₗ (for example as shown in FIG. 7A), when compared to a beam with similar proportions to that of the sum of the primary frame member 84 and secondary frame member 85. In other examples, such as that shown in Fig. 5A, the overlapping section 88 can extend along the proximal ends 81D and 81E. This configuration maintains parallelism between members 81A and 81B by increasing bending force along 81D and 81E at the proximal end of the loop assembly 80, while providing reduced bending along members 81A and 81B. Alternatively, the overlapping section 88 can extend along the entire length 81, 82 of the loop assembly 80.

In some examples, illustrated in FIGs. 6E-6G, the primary frame member 84 can be formed by a nitinol ribbon wire strip and the secondary frame member 85 can be formed by a nitinol base having a thickness different than the nitinol ribbon wire strip. The nitinol ribbon wire strip forming the primary frame member 84 and can have a thickness of about 0.002 inches, and the nitinol base forming the secondary frame member 85 can have a thickness of about 0.005 inches.

In some examples, the nitinol ribbon wire strip forming the primary frame member 84 can have a thickness of between about 25% to about 30% of a thickness of the loop assembly 80 in the overlapping section, and the nitinol base forming the secondary frame member 85 can have a thickness of between about 70% to about 75% of the thickness of the loop assembly 80 in the overlapping section.

In some examples, the loop assembly can further include an elongated shaft 9 having a proximal portion 12 and a distal portion 14A. The elongated shaft 9 can be configured to be manipulated at the proximal portion 12 to position the distal portion 14 into a heart of a patient. The elongated shaft 9 can extend along the longitudinal axis L-L, and the primary frame member 84 can be attached to the secondary frame member 85 solely via shrink sleeving.

FIG. 6A illustrates that a majority of the length of the parallel segments 82A, 82B of the symmetric support frame 182 can have a wider cross section I-I as illustrated in FIG. 6C compared to the narrower cross section II-II of the connecting segment 82C. The parallel segments 82A, 82B can include a tapering transition as illustrated in FIG. 6D and indicated in FIG. 6A that tapers from the wider width of the wider cross section I-I to the narrower width II-II of the narrower cross section II-II. A distal portion of each parallel segment 82A, 82B distal to the tapering transition can have the narrower cross section I-I. The proximal segments 82E, 83D can have a cross section I-I of approximately the same dimensions as the majority of the length of the parallel segments 82A, 82B. The symmetric support frame 182 can further include a narrower width sections 82F, 82G that respectively include a proximal portion of a respective parallel segment 82A, 82B and a distal portion of a respective proximal segment 82D, 82E. These narrower width sections 82F, 82G can have a cross section II-II as illustrated in FIG. 6B. These narrower width sections 82F, 82G can have a length of approximately 0.102 inches (2.6 millimeters). The symmetric support frame 82 can have a width of approximately 0.294 inches (7.5 millimeters) as measured between outer edges of the parallel segments 82A, 82B in the plane of the support frame 82 as illustrated.

As illustrated in FIG. 6B showing a cross sectional view of FIG. 6A taken at A-A, in some examples, the narrower cross-sectional regions II-II can have a cross sectional shape that is substantially rectangular or square with a width in the plane of the support frame 182 of about 0.005 inches (0.13 millimeters) and a height orthogonal to the plane of the support frame 182 of about 0.005 inches (0.13 millimeters).

As illustrated in FIG. 6C showing a cross sectional view of FIG. 6A taken at B-B, in some examples, the wider cross-sectional regions I-I can have a substantially rectangular cross-sectional shape with a width in the plane of the support frame 182 of about 0.01 inches (0.25 millimeters) and a height orthogonal to the plane of the support frame 182 of about 0.005 inches (0.13 millimeters).

As illustrated in FIG. 6D showing a detailed section view of FIG. 6A as indicated by D-D, in some examples, there can be a smooth transition between wider and narrower cross-sections at a knuckle 87

As mentioned previously, at least a portion of each of the spine members can include longitudinally extending first 84 and second 85 layers, the first layer 84 of the first support frame 1 being configured to slide in at least a first dimension relative to the second layer 85 of the first support frame 1.

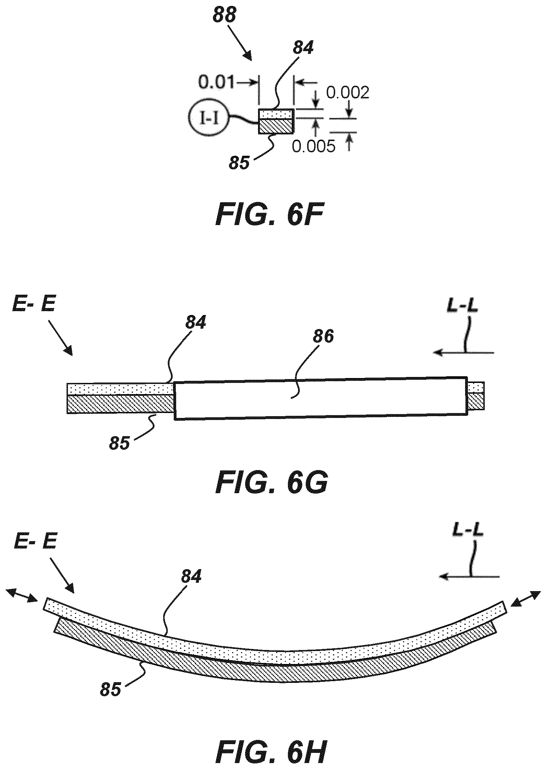

FIGs. 6E, FIG. 6G, and FIG. 6H show cross sectional views of FIG. 6A taken at E-E. Fig. 6 As illustrated in FIGs. 6E and 6G, in some examples, at least a portion of each of the spine members of each of the support frames 1A-E, 2A-E, 3A-E can include the longitudinally extending first 84 and second 85 layers. The first layer 84 of each of the support frames can be configured to longitudinally slide relative to the second layer 85 of the respective support frame. The first layer 84 of each of the support frames can include a wire strip formed of a first material, and the second layer 85 of each of the support frames can include a base formed of the first material and having a thicker cross-section I-I than a respective cross-section of the wire strip. In some examples, the loop assembly can include a sleeve 86 disposed around at least one of the primary 84 and secondary 85 frame members. The sleeve 86 can function to hold the primary 84 and secondary 85 frame members together while allowing them to slide therethrough in relation to each other in the first dimension. FIG. 6G shows the segment 82A surrounded by sleeve 86. As shown in FIG. 6H, the primary frame member 84 can be configured to slide along the secondary frame member 85 when bent. Similarly, the secondary frame member 85 can be configured to slide along the primary frame member 85 when bent.

In some examples, the first material is nitinol, and the nitinol wire strip forming the first layer 84 of each support frame can have a thickness of between about 25% to about 30% of a thickness of the respective support frame. The nitinol base forming the second layer 85 of each support frame can have a thickness of between about 70% to about 75% of the thickness of the respective support frame. More specifically, the thickness of the nitinol wire strip can measure about 0.002 inches, and the thickness of the nitinol base can measure about 0.005 inches.

In some examples, the first layer 84 of each of the support frames 1A-E, 2A-E, 3A-E is confined to the spine members 1A and 1B, 2A and 2B, 3A and 3B of the respective support frame while the second layer 85 of each of the support frames is formed of an integral nitinol base layer extending throughout the spine members and the arcuate member of the respective support frame.

In some examples, the first layer 84 of each of the support frames can extend an entire length of the spine members of the respective support frame in an unconstrained configuration, and the first layer 84 of each of the support frames extends only a portion of the entire length of the spine members of the respective support frame when the plurality of loop members 1, 2, 3 are deflected at the angle relative to the longitudinal axis L-L.

In some examples, the first layer 84 of each of the support frames being attached to the second layer 85 of the respective support frame solely via shrink sleeving. The shrink sleeving can be configured to permit the relative translation of the first layer 84 and the second layer 85 as described herein. In some examples, the shrink sleeving can include applications of surface finishes or lubricants that reduce friction between the first and second layers 84, 85 and the shrink tubing.

In some examples, there can be provided a liquid, solid, or powdered lubricant between the first layer 84 and the second layer 85 to facilitate the relative translation of the first layer 84 and the second layer 85 as described herein, thus enhancing the longitudinal flexibility of the end effector and loop assembly.

In some examples, the first layer 84 and the second layer 85 can include, at various points along their respective lengths, engagement structures such as bumps or tongue-and-groove-type fixtures which engage when the end effector 100 is flexed past a certain point (for example, see FIG. 3A) and thus prevent, slow, or inhibit further flexion of the end effector. The engagement structures can also be configured to produce progressive flexion inhibition depending on the length with which the first layer 84 is displaced from the second layer 85. Similar structures having similar purpose can also be disposed in the shrink sleeving, namely in its interior and along its length.

Reference is now made to FIG. 7A-7D. The end effector 100 for use with a catheter shaft 9 can include a plurality of loop members 1, 2, 3. Each loop member can have a support frame 1A-E, 2A-E, 3A-E including a primary frame member 84 having at least a first cross-sectional area II-II and a secondary frame member 85 having at least a second cross-sectional area I-I different from the first cross-sectional area II-II, the secondary frame member being disposed on a portion 81A, 82A of the primary frame member 84 and constrained to slide along a portion 81A, 82A of the first frame member 1. The primary frame member 84 can define a pair of proximal ends 81D and 81E, 82D and 82E and a closed distal end 81C, 82C spaced apart along a longitudinal axis L-L.

In some examples, when the end effector 100 is expanded to an unconstrained configuration, each support frame 81, 82, 83 has a pair of longitudinal side sections 81A, 81B, 82A, 82B, 83A, 83B extending between the respective pair of the proximal ends 81D and 81E, 82D and 82E, 83D and 83E and the respective distal end, and a top curved section 81C, 82C, 83C connects the pair of longitudinal side sections proximate the respective distal end. The loop member 1, 2, 3 has a lateral stiffness Sₗₐₜₑᵣₐₗ such that the longitudinal side sections 81A, 81B, 82A, 82B, 83A, 83B are configured to resist lateral movement perpendicular to the longitudinal axis L-L, the lateral stiffness Sₗₐₜₑᵣₐₗ being substantially equal to that of an alternative loop member not shown having a lone unitary layer of a first thickness in place of the primary frame member 84 and secondary frame member 85 that combined have the first thickness. The loop member has a bending stiffness S_{bend} such that the longitudinal side sections 81A, 81B, 82A, 82B, 83A, 83B are configured to resist bowing along a longitudinal length of each of the longitudinal side sections 81A, 81B, 82A, 82B, 83A, 83B when the distal end 81C, 82C, 83C contacts an obstruction, the bending stiffness S_{bend} being lower than that of the alternative loop member.

In some examples, the plurality of loop members 1, 2, 3 includes three loop members 1, 2, 3 overlapping at a common distal vertex 50 along a longitudinal axis L-L. Each primary frame member 84 of the three loop members 1, 2, 3 is affixed to a distal portion 14 of an elongated shaft 9 at each end of the respective pair of ends 81D and 81E, 82D and 82E, 83D and 83E of the respective loop member 1, 2, 3. Each of the respective support frames 81, 82, 83 can include a respective cross-sectional shape orthogonal to a respective looped path defined by each respective support frame 81, 82, 83, with each of the respective cross-sectional shapes varying along each respective support frame 81, 82, 83.

In some examples, each of the plurality of loop members 1, 2, 3 can include an inner tubular housing 1A, 2A, 2B surrounding at least a portion of the respective support frame 1, 2, 3, an outer tubular housing 1B, 2B, 3B surrounding at least a portion of the inner tubular housing 1A, 2A, 2B and bonded to the inner tubular housing 1A, 2A, 2B, and a plurality of electrical conductors 37 disposed at least partially within the outer tubular housing 1B, 2B, 3B and outside of the inner tubular housing 1A, 2A, 2B.

In some examples, each of the respective support frames 1, 2, 3 can include a respective pair of parallel segments 81A and 81B, 82A and 82B, 83A and 83B, each segment in the respective pair of parallel segments having a respective length 81A and 81B, 82A and 82B, 83A and 83B. A majority of the respective length of each segment in each of the respective pairs of parallel segments can be approximately coplanar with a majority of the respective length of each of the other segments in each of the respective pairs of parallel segments when the end effector 100 is in a flattened configuration. The majority of the respective lengths the segments of at least one of the respective pairs of parallel segments can be non-coplanar with the majority of the respective length of at least one segment of the other respective pairs of parallel segments when the end effector 100 is in an unconstrained configuration.

In some examples, the plurality of loop members 1, 2, 3 can overlap at a common distal vertex 50 along the longitudinal axis L-L. The end effector 100 can further include a mechanical linkage 50 binding the plurality of loop members 1, 2, 3 at the distal vertex. The mechanical linkage 50 can include at least one of: a rectangular or ovular shape having an opening through which the plurality of loop members 1, 2, 3 extend and a side having a seam, a rectangular or ovular shape having an opening through which the plurality of loop members 1, 2, 3 extend and four contiguous sides, a rectangular or ovular shape having three openings each having a respective loop member of the plurality of loop members 1, 2, 3 extending therethrough, a cylindrical shape having plurality of passageways therethrough, the plurality of passageways each having a respective loop member of the plurality of loop members 1, 2, 3 extending therethrough, and a tapered ring having an annular opening through which the plurality of loop members 1, 2, 3 extend and a tapered height extending across a diameter of annular opening. A plurality of electrodes 37 can be affixed to the plurality of loop members 1, 2, 3, each electrode of the plurality of electrodes 37 having a surface characterized by roughness parameter *Ra* representing an arithmetical mean deviation of a profile of the surface, where *Ra* measures from about 0.3 micrometers to about 0.4 micrometers. The end effector can further include at least one pull wire extending through an elongated shaft 9 and attached to a distal portion 14 of the elongated shaft 9 so that when the pull wire is retracted toward a proximal portion 12 relative to the elongated shaft 9, the distal portion 14 of the elongated shaft 9 and the end effector 100 are bent at an angle with respect to the longitudinal axis L-L.

FIGs. 7A-7D are illustrations of deformation of support frame assemblies as a result of application of various forces. Each loop assembly 80 includes a first support frame 81, a second support frame 82, and a third support frame 83. The first and third support frames 81, 83 are outer support frames surrounding the second central support frame 82. The loop assembly 80 includes a mechanical linkage 50 joining the first, second, and third support frames 81, 82, 83 at the distal vertex.

FIG. 7A illustrates the first support frame 81 pressed into a planar surface S as an apex of a wedge W is pressed with a force F1 into the third support frame 83. The loop assembly 80 is aligned approximately orthogonal to the planar surface S. The force F1 is applied approximately orthogonal to, and in a direction toward the planar surface S. The force F1 is applied centrally along the length of the outer parallel segment 83B of the third support frame 83. The outer parallel segment 83B of the third support frame bows toward the surface S as a result of the force F1. The loop assembly 80 can be configured to resist spine-to-spine contact when the force F1 is applied. In FIG. 7A, the outer parallel segment 83B of the third support frame is bowed to contact the second support frame 82. The force F1 sufficient to move the outer parallel segment 83B of the third support frame 83 into contact with the second support frame 82 can be a metric used to compare variations in loop assembly design (e.g. with differing support frame cross section design) where a higher force F1 indicates a more favorable result for this test.

FIG. 7B illustrates the loop assembly 80 compressed between two planar surfaces S1, S2 with a force F2. The planar surfaces S1, S2 are aligned approximately parallel to each other. The loop assembly 80 is aligned approximately orthogonal to the planar surfaces S1, S2. An outer parallel segment 81A of the first support frame 81 is pressed by the first planar surface S1. The outer parallel segment 83B of the third support frame 83 is pressed by the second planar surface S2. As a result of the compression by the force F2 between the planar surfaces S1, S2, the outer parallel segments 81A, 83B bow toward the opposite planar surface S1, S2. The loop assembly 80 can be configured to resist spine-to-spine contact when the force F2 is applied. The force F2 sufficient to move the outer parallel segment 83B of the third support frame 83 or the outer parallel segment 81A of the first support frame 81 into contact with the second support frame 82 can be a metric used to compare variations in loop assembly design (e.g. with differing support frame cross section design) where a higher force F2 indicates a more favorable result for this test. In the preferred embodiments, F1 or F2 is approximately 10 gram-force or greater. The range of suitable force is about 10 gram-force to 50 gram-force for F1 (as shown in FIG. 7A) and 10 gram-force to 70 gram-force for F2 (as shown in FIG. 7B).

FIG. 7C illustrates the loop assembly 80 compressed by a planar surface S applying a force F3 to a distal end of the loop assembly 80. The distal portion 14A of the shaft 9 is held at a position in the proximal direction of the connector tubing 46 such that the longitudinal axis L-L defined by the shaft 9 is approximately orthogonal to the surface S. The force F3 is applied to the connector segments 81C, 83C of the first and third support frames 81, 83. As a result of the compression by the force F3 in the direction of the longitudinal axis L-L, the loop assembly 80 and connector tubing 46 deflect out of alignment with the longitudinal axis L-L. This is to ensure the distal section 14A of shaft 9 and/or end effector are sufficiently flexible so that the end effector and/or distal section of the shaft buckles at a sufficiently low force when applied to a heart wall to inhibit the end effector from puncturing the heart wall.

FIG. 7D illustrates the loop assembly 80 deflected by a force F4 applied approximately orthogonal to the loop assembly near the mechanical linkage 50 at the distal vertex. The distal portion 14A of the shaft 9 is held near the connector tubing 46. As a result of the force F4, the support frame 80 is deflected out of alignment with the longitudinal axis L-L. In some use cases, it can be desired to have a greater or lesser deflection as a result of Force F4 depending on the geometry of a treatment area and/or preferences of a physician regarding tactile feedback.

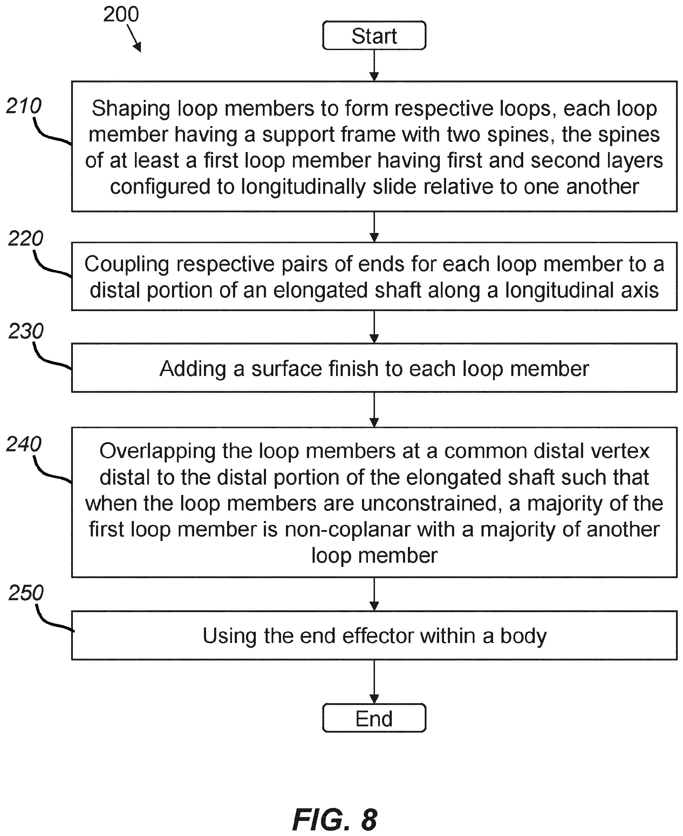

Reference is made to FIG. 8 showing a method 200 in accordance with the disclosed technology. The method 200 can include shaping step 210 at least a first loop member and a second loop member to each form a respective loop, each of the first and second loop members having a support frame having two spines, the spines of at least the first loop member each having a first layer extending longitudinally along a first length of the respective spine and a second layer extending longitudinally along a second length of the respective spine overlapping the first length, and first layer being configured to longitudinally slide relative to the second layer of each respective spine. In some examples, shaping at least the first loop member and the second loop member further includes configuring the spines of the second loop member to each have the first layer extending longitudinally along the first length of the respective spine and the second layer extending longitudinally along the second length of the respective spine overlapping the first length, with first layer being configured to longitudinally slide relative to the second layer of each respective spine.

The method 200 can further include a coupling step 220 a respective pair of ends for each of the at least first and second loop members to a distal portion of an elongated shaft extending along a longitudinal axis L-L.

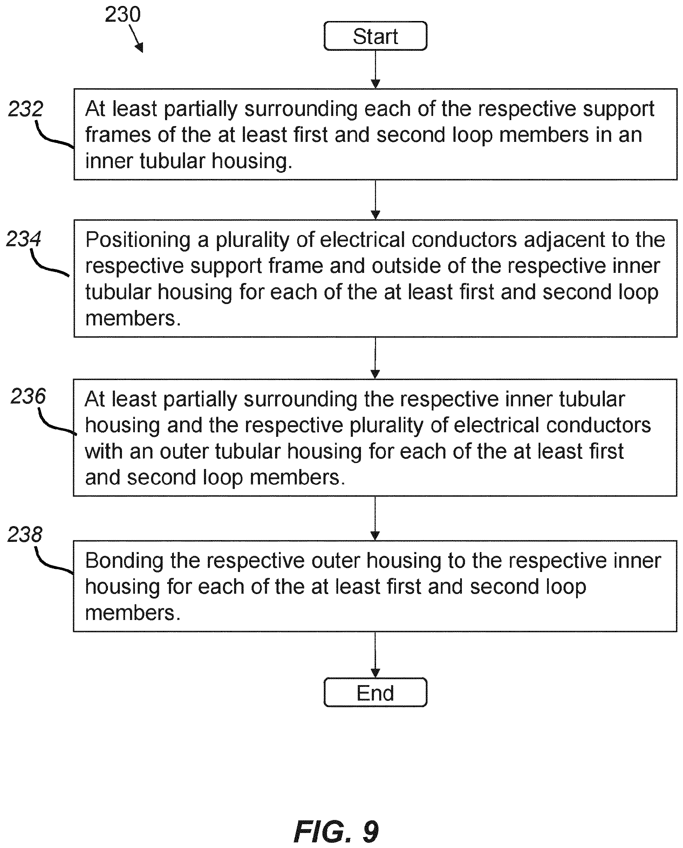

The method 200 can also include a step 230 of adding a surface finish to each loop member, which may involve multiple sub-steps as described in more detail with respect to FIG. 9. In some examples, adding a surface finish step 230 can further include at least partially surrounding 232 each of the respective support frames of the at least first and second loop members in an inner tubular housing, and a positioning 234 a plurality of electrical conductors adjacent to the respective support frame and outside of the respective inner tubular housing for each of the at least first and second loop members. Adding a surface finish 230 can further include at least partially surrounding 236 the respective inner tubular housing and the respective plurality of electrical conductors with an outer tubular housing for each of the at least first and second loop members, and bonding 238 the respective outer tubular housing to the respective inner tubular housing for each of the at least first and second loop members and affixing the respective support frames of the at least first and second loop members to the distal portion of the elongated shaft. In some examples, the method 200 can further include affixing a plurality of electrodes to the at least first and second loop members, abrading at least a portion of a surface of each electrode of the plurality of electrodes, and swaging each electrode of the plurality of electrodes.

The method 200 can further include overlapping step 240 the at least first and second loop members at a common distal vertex distal to the distal portion of the elongated shaft such that when the at least first and second loop members are unconstrained, a majority of the first loop member is non-coplanar with a majority of the second loop member.

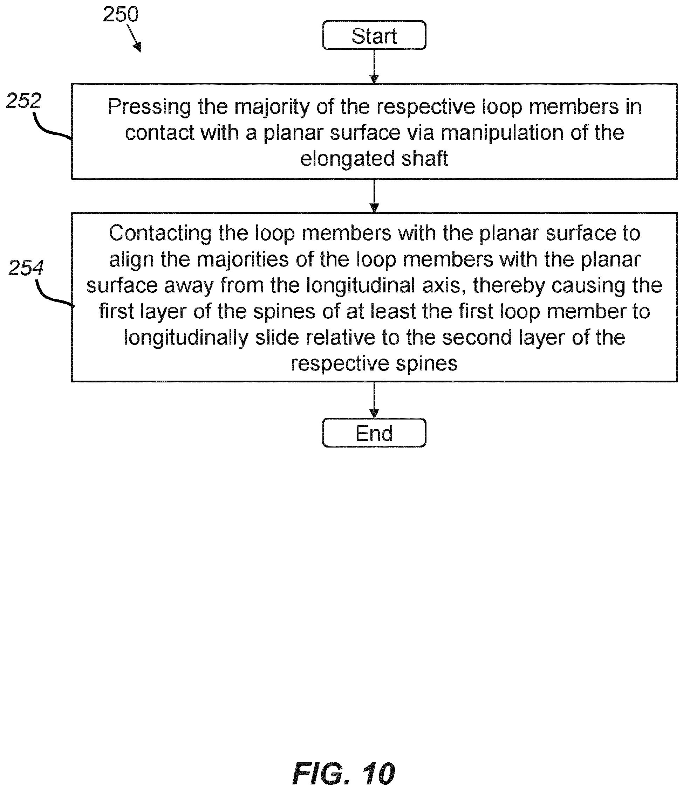

The method 200 can also include a use step 250 that involves using the end effector (e.g., end effector 100) within a body. For example, as shown in FIG. 10, use step 250 may include pressing 252 the majority of the first loop member and the majority of the second loop member in contact with a planar surface S via manipulation of the elongated shaft, and contacting 254 the at least first and second loop members with the planar surface S to align the majorities of the at least first and second loop members with the planar surface S away from the longitudinal axis L-L, thereby causing the first layer of the spines of at least the first loop member to longitudinally slide relative to the second layer of the respective spines. In some examples, contacting the at least first and second loop members with the planar surface S further includes causing the first layer of the spines of the second loop member to longitudinally slide relative to the second layer of the respective spines. In some examples, the method 200 can further include positioning the at least first and second loop members and distal portion of the elongated shaft within an intravascular catheter while a proximal portion of the elongated shaft extends proximally from the intravascular catheter, moving the at least first and second loop members out of a distal end of the catheter via manipulation of the proximal portion of the elongated shaft, and pressing the majorities of the at least first and second loop members in contact with the planar surface S via manipulation of the proximal portion of the elongated shaft.

As will be appreciated, the method 200 just described can be varied in accordance with the various elements and implementations described herein. That is, methods in accordance with the disclosed technology can include all or some of the steps described above and/or can include additional steps not expressly disclosed above. Further, methods in accordance with the disclosed technology can include some, but not all, of a particular step described above. Further still, various methods described herein can be combined in full or in part. That is, methods in accordance with the disclosed technology can include at least some elements or steps of a first method and at least some elements or steps of a second method.

The invention is as defined in the appended claims.

## Claims

1. An end effector for use with a catheter shaft, the end effector comprising:
a plurality of loop members, each loop member having a support frame comprising:
a primary frame member (84) having at least a first cross-sectional area, the primary frame member defining a pair of proximal ends (81D, 81E, 82D, 82E) and a closed distal end (81C, 82C) spaced apart along a longitudinal axis; and
**characterized by** a secondary frame member (85) having at least a second cross-sectional area different from the first cross-sectional area, the secondary frame member being disposed on a portion of the primary frame member and constrained to slide along a portion of the primary frame member.

2. The end effector of claim 1,
when the end effector is expanded to an unconstrained configuration, each support frame comprising a pair of longitudinal side sections extending between the respective pair of the proximal ends and the respective distal end, and a top curved section connecting the pair of longitudinal side sections proximate the respective distal end;
the loop member having a lateral stiffness such that the longitudinal side sections are configured to resist lateral movement perpendicular to the longitudinal axis, the lateral stiffness being substantially equal to that of an alternative loop member having a lone unitary layer of a first thickness in place of the primary frame member and secondary frame member that combined have the first thickness; and
the loop member having a bending stiffness such that the longitudinal side sections are configured to resist bowing along a longitudinal length of each of the longitudinal side sections when the distal end contacts an obstruction, the bending stiffness being lower than that of the alternative loop member.

3. The end effector of claim 1,
the plurality of loop members comprising three loop members overlapping at a common distal vertex along a longitudinal axis;
each primary frame member of the three loop members being affixed to a distal portion of an elongated shaft at each end of the respective pair of ends of the respective loop member; and
each of the respective support frames comprising a respective cross-sectional shape orthogonal to a respective looped path defined by each respective support frame, with each of the respective cross-sectional shapes varying along each respective support frame.

4. The end effector of claim 1, each of the plurality of loop members comprising:
an inner tubular housing surrounding at least a portion of the respective support frame;
an outer tubular housing surrounding at least a portion of the inner tubular housing and bonded to the inner tubular housing; and
a plurality of electrical conductors disposed at least partially within the outer tubular housing and outside of the inner tubular housing.

5. The end effector (100) of claim 1, each of the respective support frames comprising:
a respective pair of parallel segments, each segment in the respective pair of parallel segments comprising a respective length;
a majority of the respective length of each segment in each of the respective pairs of parallel segments being approximately coplanar with a majority of the respective length of each of the other segments in each of the respective pairs of parallel segments when the end effector is in a flattened configuration; and
the majority of the respective lengths the segments of at least one of the respective pairs of parallel segments being non-coplanar with the majority of the respective length of at least one segment of the other respective pairs of parallel segments when the end effector is in an unconstrained configuration.

6. The end effector of claim 1,
the plurality of loop members overlapping at a common distal vertex along the longitudinal axis,
the end effector further comprising:
a mechanical linkage binding the plurality of loop members at the distal vertex, the mechanical linkage comprising at least one of:
a rectangular or ovular shape comprising an opening through which the plurality of loop members extend and a side comprising a seam;
a rectangular or ovular shape comprising an opening through which the plurality of loop members extend and four contiguous sides;
a rectangular or ovular shape comprising three openings each comprising a respective loop member of the plurality of loop members extending therethrough;
a cylindrical shape comprising plurality of passageways therethrough, the plurality of passageways each comprising a respective loop member of the plurality of loop members extending therethrough; and
a tapered ring comprising an annular opening through which the plurality of loop members extend and a tapered height extending across a diameter of annular opening;
a plurality of electrodes affixed to the plurality of loop members, each electrode of the plurality of electrodes comprising a surface **characterized by** roughness parameter *Ra* representing an arithmetical mean deviation of a profile of the surface, where *Ra* measures from about 0.3 micrometers to about 0.4 micrometers; and
at least one pull wire extending through an elongated shaft and attached to a distal portion of the elongated shaft so that when the pull wire is retracted toward a proximal portion relative to the elongated shaft, the distal portion of the elongated shaft and the end effector are bent at an angle with respect to the longitudinal axis.

7. The end effector of claim 1, the secondary frame member being configured to slide in at least a first dimension relative to the primary frame member; and each loop member further comprising:
a sleeve disposed around the at least one of the primary and secondary frame members.

8. The end effector of claim 7,
the first dimension being longitudinal;
the primary frame member comprising a metallic wire strip and longitudinally extending along at least a first portion of a length of the loop assembly; and
the secondary frame member comprising a metallic base and longitudinally extending along at least a second portion of the length of the loop assembly, the first portion of the length of the loop assembly at least partially overlapping the second portion of the length of the loop assembly at an overlapping section.

9. The end effector of claim 8,
the primary frame member being formed by a nitinol ribbon wire strip; and
the secondary frame member being formed by a nitinol base having a greater thickness than the nitinol ribbon wire strip.

10. The end effector of claim 9,
(a) the nitinol ribbon wire strip forming the primary frame member having a thickness of about 0.002 inches; and
the nitinol base forming the secondary frame member having a thickness of about 0.005 inches; or
(b) the nitinol ribbon wire strip forming the primary frame member having a thickness of between about 25% to about 30% of a thickness of the loop assembly in the overlapping section; and
the nitinol base forming the secondary frame member having a thickness of between about 70% to about 75% of the thickness of the loop assembly in the overlapping section.

11. The end effector of claim 7, further comprising:
an elongated shaft comprising a proximal portion and a distal portion, the elongated shaft configured to be manipulated at the proximal portion to position the distal portion into a heart of a patient, the elongated shaft extending along the longitudinal axis, and
the primary frame member being attached to the secondary frame member solely via shrink sleeving.

12. An assembly for a mapping catheter comprising:
a tubular member extending along a longitudinal axis;
the end effector of claim 1, each support frame affixed to a distal portion of the tubular member, each support frame extending through the respective loop member to define two spine members extending from the tubular member and connected to an arcuate member; and
each loop member further comprising: a plurality of electrodes disposed on the respective support frame;
at least a portion of each of the spine members comprising longitudinally extending the primary and secondary frame members, the primary frame member being configured to slide in at least a first dimension relative to the secondary frame member.

13. The assembly of claim 12,
the first dimension being longitudinal;
a first loop member of the plurality of loop members being arrayed on a first plane;
a second loop member of the plurality of loop members being arrayed on a second plane that intersects with the first plane; and
a third loop member of the plurality of loop members being arrayed on a third plane that intersects with the first plane and the second plane such that a majority of a length of each of the three loop members is non-coplanar with the majority of the length of at least one of the other three loop members in an unconstrained configuration.

14. The assembly of claim 12,
at least a portion of each of the spine members of each of the support frames comprising the longitudinally extending primary and secondary frame members, the primary frame member of each of the support frames being configured to longitudinally slide relative to the secondary frame member of the respective support frame;
the primary frame member of each of the support frames comprising a wire strip formed of a first material; and
the secondary frame member of each of the support frames comprising a base formed of the first material and having a thicker cross-section than a respective cross-section of the wire strip.

15. The assembly of claim 14,
the first material comprising nitinol;
the nitinol wire strip forming the primary frame member of each support frame having a thickness of between about 25% to about 30% of a thickness of the respective support frame; and
the nitinol base forming the secondary frame member of each support frame having a thickness of between about 70% to about 75% of the thickness of the respective support frame, optionally the thickness of the nitinol wire strip measuring about 0.002 inches; and
the thickness of the nitinol base measuring about 0.005 inches.

16. The assembly of claim 14,
the primary frame member of each of the support frames being confined to the spine members of the respective support frame; and
the secondary frame member of each of the support frames being formed of an integral nitinol base layer extending throughout the spine members and the arcuate member of the respective support frame.

17. The assembly of claim 14,
the primary frame member of each of the support frames extending an entire length of the spine members of the respective support frame in an unconstrained configuration; and
the primary frame member of each of the support frames extending only a portion of the entire length of the spine members of the respective support frame when the plurality of loop members are deflected at an angle relative to the longitudinal axis.

18. The assembly of claim 14,
the primary frame member of each of the support frames being attached to the secondary frame member of the respective support frame solely via shrink sleeving.

## Patentansprüche

1. Endeffektor für die Verwendung mit einem Katheterschaft, der Endeffektor umfassend:
eine Vielzahl von Schleifenelementen, wobei jedes Schleifenelement einen Stützrahmen aufweist, der umfasst:
ein primäres Rahmenelement (84) mit mindestens einer ersten Querschnittsfläche, wobei das primäre Rahmenelement ein Paar proximaler Enden (81D, 81E, 82D, 82E) und ein geschlossenes distales Ende (81C, 82C) definiert, die entlang einer Längsachse beabstandet sind; und
**gekennzeichnet durch** ein sekundäres Rahmenelement (85) mit mindestens einer zweiten Querschnittsfläche, die sich von der ersten Querschnittsfläche unterscheidet, wobei das sekundäre Rahmenelement auf einem Abschnitt des primären Rahmenelements angeordnet ist und darauf beschränkt ist, entlang eines Abschnitts des primären Rahmenelements zu gleiten.

2. Endeffektor nach Anspruch 1,
wenn der Endeffektor in eine unbeschränkte Konfiguration expandiert ist, jeder Stützrahmen ein Paar Längsseitenabschnitte umfasst, die sich zwischen dem jeweiligen Paar proximaler Enden und dem jeweiligen distalen Ende erstrecken, sowie einen oberen gekrümmten Abschnitt, der das Paar Längsseitenabschnitte in der Nähe des jeweiligen distalen Endes verbindet;
wobei das Schleifenelement eine solche laterale Steifigkeit aufweist, dass die Längsseitenabschnitte konfiguriert sind, um einer lateralen Bewegung senkrecht zur Längsachse standzuhalten, wobei die laterale Steifigkeit im Wesentlichen der eines alternativen Schleifenelements entspricht, das anstelle des primären Rahmenelements und des sekundären Rahmenelements, die zusammen die erste Dicke aufweisen, eine einzelne einheitliche Schicht einer ersten Dicke aufweist; und
wobei das Schleifenelement eine solche Biegesteifigkeit aufweist, dass die Längsseitenabschnitte konfiguriert sind, um einem Verbiegen entlang einer Längslänge jedes der Längsseitenabschnitte standzuhalten, wenn das distale Ende ein Hindernis berührt, wobei die Biegesteifigkeit kleiner ist als die des alternativen Schleifenelements.

3. Endeffektor nach Anspruch 1,
wobei die Vielzahl von Schleifenelementen drei Schleifenelemente umfasst, die sich an einem gemeinsamen distalen Scheitelpunkt entlang einer Längsachse überlappen;
wobei jedes primäre Rahmenelement der drei Schleifenelemente an einem distalen Abschnitt eines länglichen Schafts an jedem Ende des jeweiligen Paars von Enden des jeweiligen Schleifenelements befestigt ist; und
jeder der jeweiligen Stützrahmen eine jeweilige Querschnittsform umfasst, die orthogonal zu einem jeweiligen Schleifenpfad ist, der von jedem jeweiligen Stützrahmen definiert wird, wobei jede der jeweiligen Querschnittsformen entlang jedes jeweiligen Stützrahmens variiert.

4. Endeffektor nach Anspruch 1, wobei jedes der Vielzahl von Schleifenelementen umfasst:
ein inneres röhrenförmiges Gehäuse, das mindestens einen Abschnitt des jeweiligen Stützrahmens umgibt;
ein äußeres röhrenförmiges Gehäuse, das mindestens einen Abschnitt des inneren röhrenförmigen Gehäuses umgibt und an das innere röhrenförmige Gehäuse gebunden ist; und
eine Vielzahl von elektrischen Leitern, die mindestens teilweise innerhalb des äußeren röhrenförmigen Gehäuses und außerhalb des inneren röhrenförmigen Gehäuses angeordnet ist.

5. Endeffektor (100) nach Anspruch 1, wobei jeder der jeweiligen Stützrahmen umfasst:
ein jeweiliges Paar paralleler Segmente, wobei jedes Segment im jeweiligen Paar paralleler Segmente eine jeweilige Länge aufweist;
wobei ein Großteil der jeweiligen Länge jedes Segments in jedem der jeweiligen Paare paralleler Segmente ungefähr koplanar mit einem Großteil der jeweiligen Länge jedes der anderen Segmente in jedem der jeweiligen Paare paralleler Segmente ist, wenn sich der Endeffektor in einer abgeflachten Konfiguration befindet; und
wobei der Großteil der jeweiligen Längen der Segmente von mindestens einem der jeweiligen Paare paralleler Segmente nicht koplanar mit dem Großteil der jeweiligen Länge von mindestens einem Segment der anderen jeweiligen Paare paralleler Segmente ist, wenn sich der Endeffektor in einer unbeschränkten Konfiguration befindet.

6. Endeffektor nach Anspruch 1,
wobei sich die Vielzahl der Schleifenelemente an einem gemeinsamen distalen Scheitelpunkt entlang der Längsachse überlappt,
wobei der Endeffektor ferner umfasst:
eine mechanische Verbindung, die die Vielzahl von Schleifenelementen am distalen Scheitelpunkt verbindet, wobei die mechanische Verbindung mindestens eines ist von:
einer rechteckigen oder ovalen Form, umfassend eine Öffnung, durch die hindurch sich die Vielzahl von Schleifenelementen erstreckt, und eine Seite, umfassend eine Naht;
einer rechteckigen oder ovalen Form, umfassend eine Öffnung, durch die hindurch sich die Vielzahl von Schleifenelementen erstreckt, und vier aneinandergrenzende Seiten;
einer rechteckigen oder ovalen Form, umfassend drei Öffnungen, jede umfassend ein jeweiliges Schleifenelement der Vielzahl von Schleifenelementen, das sich dahindurch erstreckt;
einer zylindrischen Form, umfassend die Vielzahl von Durchgängen dahindurch, die Vielzahl von Durchgängen jeweils umfassend ein jeweiliges Schleifenelement der Vielzahl von Schleifenelementen, das sich dahindurch erstreckt; und
einem konischen Ring, umfassend eine ringförmige Öffnung, durch die hindurch sich die Vielzahl von Schleifenelementen erstreckt, und eine konische Höhe, die sich über den Durchmesser der ringförmigen Öffnung hinweg erstreckt;
eine Vielzahl von Elektroden, die an der Vielzahl von Schleifenelementen befestigt ist, jede Elektrode der Vielzahl von Elektroden umfassend eine Oberfläche, **gekennzeichnet durch** einen Rauheitsparameter Ra, der eine arithmetische mittlere Abweichung eines Profils der Oberfläche darstellt, wobei Ra von etwa 0,3 Mikrometer bis etwa 0,4 Mikrometer beträgt; und
mindestens einen Zugdraht, der sich durch den länglichen Schaft hindurch erstreckt und an einem distalen Abschnitt des länglichen Schafts angebracht ist, so dass, wenn der Zugdraht in Richtung eines proximalen Abschnitts relativ zum länglichen Schaft zurückgezogen wird, der distale Abschnitt des länglichen Schafts und der Endeffektor in einem Winkel hinsichtlich der Längsachse gebogen werden.

7. Endeffektor nach Anspruch 1, wobei das sekundäre Rahmenelement konfiguriert ist, um in mindestens einer ersten Dimension relativ zum primären Rahmenelement zu gleiten; und jedes Schleifenelement ferner umfasst:
eine Hülse, die um mindestens eines der primären und sekundären Rahmenelemente angeordnet ist.

8. Endeffektor nach Anspruch 7,
wobei die erste Dimension längs verläuft;
wobei das primäre Rahmenelement einen metallischen Drahtstreifen umfasst und sich in Längsrichtung entlang mindestens eines ersten Abschnitts einer Länge der Schleifenanordnung erstreckt; und
wobei das sekundäre Rahmenelement eine metallische Basis umfasst und sich in Längsrichtung entlang mindestens eines zweiten Abschnitts der Länge der Schleifenanordnung erstreckt, wobei der erste Abschnitt der Länge der Schleifenanordnung den zweiten Abschnitt der Länge der Schleifenanordnung an einem Überlappungsabschnitt mindestens teilweise überlappt.

9. Endeffektor nach Anspruch 8,
wobei das primäre Rahmenelement durch einen Nitinol-Flachbanddrahtstreifen gebildet wird; und
das sekundäre Rahmenelement aus einer Nitinolbasis gebildet wird, die eine größere Dicke aufweist als der Nitinol-Flachbanddrahtstreifen.

10. Endeffektor nach Anspruch 9,
(a) der Nitinol-Flachbanddrahtstreifen, der das primäre Rahmenelement bildet, eine Dicke von etwa 0,002 Zoll aufweist; und
die Nitinolbasis, die das sekundäre Rahmenelement bildet, eine Dicke von etwa 0,005 Zoll aufweist; oder
(b) der Nitinol-Flachbanddrahtstreifen, der das primäre Rahmenelement bildet, eine Dicke zwischen etwa 25 % und etwa 30 % der Dicke der Schleifenanordnung im Überlappungsabschnitt aufweist; und
wobei die Nitinolbasis, die das sekundäre Rahmenelement bildet, eine Dicke von zwischen etwa 70 % bis etwa 75 % der Dicke der Schleifenanordnung im Überlappungsabschnitt aufweist.

11. Endeffektor nach Anspruch 7, ferner umfassend:
einen länglichen Schaft, der einen proximalen Abschnitt und einen distalen Abschnitt umfasst, wobei der längliche Schaft konfiguriert ist, um am proximalen Abschnitt manipuliert zu werden, um den distalen Abschnitt in einem Herzen eines Patienten zu positionieren, wobei sich der längliche Schaft entlang der Längsachse erstreckt, und
wobei das primäre Rahmenelement ausschließlich über Schrumpfschläuche an dem sekundären Rahmenelement angebracht wird.

12. Anordnung für einen Mapping-Katheter, umfassend:
ein röhrenförmiges Element, das sich entlang einer Längsachse erstreckt;
den Endeffektor nach Anspruch 1, wobei jeder Stützrahmen an einem distalen Abschnitt des röhrenförmigen Elements befestigt ist und sich jeder Stützrahmen durch das jeweilige Schleifenelement erstreckt, um zwei Rückgratelemente zu definieren, die sich vom röhrenförmigen Element aus erstrecken und mit einem bogenförmigen Element verbunden sind; und
wobei jedes Schleifenelement ferner umfasst: eine Vielzahl von Elektroden, die auf dem jeweiligen Stützrahmen angeordnet ist;
wobei mindestens ein Abschnitt jedes der Rückgratelemente die sich in Längsrichtung erstreckenden primären und sekundären Rahmenelemente umfasst, wobei das primäre Rahmenelement konfiguriert ist, um in mindestens einer ersten Dimension relativ zum sekundären Rahmenelement zu gleiten.

13. Anordnung nach Anspruch 12,
wobei die erste Dimension längs verläuft;
ein erstes Schleifenelement der Vielzahl von Schleifenelementen auf einer ersten Ebene im Array angeordnet ist;
ein zweites Schleifenelement der Vielzahl von Schleifenelementen auf einer zweiten Ebene, die die erste Ebene schneidet, im Array angeordnet ist; und
ein drittes Schleifenelement der Vielzahl von Schleifenelementen auf einer dritten Ebene im Array angeordnet ist, die sich mit der ersten Ebene und der zweiten Ebene schneidet, so dass ein Großteil einer Länge jedes der drei Schleifenelemente nicht koplanar mit dem Großteil der Länge von mindestens einem der anderen drei Schleifenelemente in einer uneingeschränkten Konfiguration ist.

14. Anordnung nach Anspruch 12,
wobei mindestens ein Abschnitt jedes der Rückgratelemente jedes der Stützrahmen die sich in Längsrichtung erstreckenden primären und sekundären Rahmenelemente umfasst, wobei das primäre Rahmenelement jedes der Stützrahmen konfiguriert ist, um in Längsrichtung relativ zum sekundären Rahmenelement des jeweiligen Stützrahmens zu gleiten;
wobei das primäre Rahmenelement jedes der Stützrahmen einen Drahtstreifen umfasst, der aus einem ersten Material gebildet ist; und
wobei das sekundäre Rahmenelement jedes der Stützrahmen eine Basis umfasst, die aus dem ersten Material gebildet ist und einen dickeren Querschnitt als der jeweilige Querschnitt des Drahtstreifens aufweist.

15. Anordnung nach Anspruch 14,
wobei das erste Material Nitinol umfasst;
wobei der Nitinoldrahtstreifen das primäre Rahmenelement jedes Stützrahmens bildet und eine Dicke zwischen etwa 25 % bis etwa 30 % der Dicke des jeweiligen Stützrahmens aufweist; und
wobei die Nitinolbasis das sekundäre Rahmenelement jedes Stützrahmens bildet und eine Dicke zwischen etwa 70 % bis etwa 75 % der Dicke des jeweiligen Stützrahmens aufweist, wobei die Dicke des Nitinoldrahtstreifens optional etwa 0,002 Zoll beträgt; und
die Dicke der Nitinolbasis etwa 0,005 Zoll beträgt.

16. Anordnung nach Anspruch 14,
wobei das primäre Rahmenelement jedes der Stützrahmen auf die Rückgratelemente des jeweiligen Stützrahmens beschränkt ist; und
das sekundäre Rahmenelement jedes Stützrahmens aus einer integrierten Nitinol-Basisschicht gebildet ist, die sich über die gesamten Rückgratelemente und das bogenförmige Element des jeweiligen Stützrahmens erstreckt.

17. Anordnung nach Anspruch 14,
wobei sich das primäre Rahmenelement jedes Stützrahmens in einer uneingeschränkten Konfiguration über die gesamte Länge der Rückgratelemente des jeweiligen Stützrahmens erstreckt; und
wobei sich das primäre Rahmenelement jedes Stützrahmens nur über einen Abschnitt der Gesamtlänge der Rückgratelemente des jeweiligen Stützrahmens erstreckt, wenn die Vielzahl von Schleifenelementen in einem Winkel relativ zur Längsachse abgelenkt ist.

18. Anordnung nach Anspruch 14,
wobei das primäre Rahmenelement jedes Stützrahmens ausschließlich über Schrumpfschläuche an dem sekundären Rahmenelement des jeweiligen Stützrahmens angebracht ist.

## Revendications

1. Effecteur terminal pour utilisation avec une tige de cathéter, l'effecteur terminal comprenant :
une pluralité d'éléments de boucle, chaque élément de boucle ayant un châssis de support comprenant :
un élément de châssis primaire (84) ayant au moins une première aire en coupe transversale, l'élément de châssis primaire définissant une paire d'extrémités proximales (81D, 81E, 82D, 82E) et une extrémité distale fermée (81C, 82C) espacées le long d'un axe longitudinal ; et
**caractérisé par** un élément de châssis secondaire (85) ayant au moins une seconde aire en coupe transversale différente de la première aire en coupe transversale, l'élément de châssis secondaire étant disposé sur une partie de l'élément de châssis primaire et contraint de coulisser le long d'une partie de l'élément de châssis primaire.

2. Effecteur terminal selon la revendication 1,
lorsque l'effecteur terminal est expansé à une configuration sans contrainte, chaque châssis de support comprenant une paire de sections latérales longitudinales s'étendant entre la paire respective des extrémités proximales et l'extrémité distale respective, et une section incurvée supérieure reliant la paire de sections latérales longitudinales à proximité de l'extrémité distale respective ;
l'élément de boucle ayant une rigidité latérale de telle sorte que les sections latérales longitudinales sont conçues pour résister à un mouvement latéral perpendiculaire à l'axe longitudinal, la rigidité latérale étant sensiblement égale à celle d'un élément de boucle alternatif ayant une seule couche d'un seul tenant d'une première épaisseur au lieu de l'élément de châssis primaire et de l'élément de châssis secondaire qui, combinés, ont la première épaisseur ; et
l'élément de boucle ayant une rigidité en flexion de telle sorte que les sections latérales longitudinales sont conçues pour résister à un cintrage le long d'une longueur longitudinale de chacune des sections latérales longitudinales lorsque l'extrémité distale vient en contact avec une obstruction, la rigidité en flexion étant inférieure à celle de l'élément de boucle alternatif.

3. Effecteur terminal selon la revendication 1,
la pluralité d'éléments de boucle comprenant trois éléments de boucle se chevauchant au niveau d'un sommet distal commun le long d'un axe longitudinal ;
chaque élément de châssis primaire des trois éléments de boucle étant attaché à une partie distale d'une tige allongée au niveau de chaque extrémité de la paire respective d'extrémités de l'élément de boucle respectif ; et
chacun des châssis de support respectifs comprenant une forme en coupe transversale respective orthogonale à un chemin bouclé respectif défini par chaque châssis de support respectif, avec chacune des formes en coupe transversale respectives variant le long de chaque châssis de support respectif.

4. Effecteur terminal selon la revendication 1, chacun parmi la pluralité d'éléments de boucle comprenant :
un logement tubulaire interne entourant au moins une partie du châssis de support respectif ;
un logement tubulaire externe entourant au moins une partie du logement tubulaire interne et lié au logement tubulaire interne ; et
une pluralité de conducteurs électriques disposés au moins partiellement au sein du logement tubulaire externe et à l'extérieur du logement tubulaire interne.

5. Effecteur terminal (100) selon la revendication 1, chacun des châssis de support respectifs comprenant :
une paire respective de segments parallèles, chaque segment dans la paire respective de segments parallèles comprenant une longueur respective ;
une majorité de la longueur respective de chaque segment dans chacune des paires respectives de segments parallèles étant approximativement coplanaire avec une majorité de la longueur respective de chacun des autres segments dans chacune des paires respectives de segments parallèles lorsque l'effecteur terminal est dans une configuration aplatie ; et
la majorité des longueurs respectives des segments d'au moins l'une des paires respectives de segments parallèles étant non coplanaire avec la majorité de la longueur respective d'au moins un segment des autres paires respectives de segments parallèles lorsque l'effecteur terminal est dans une configuration sans contrainte.

6. Effecteur terminal selon la revendication 1,
la pluralité d'éléments de boucle se chevauchant au niveau d'un sommet distal commun le long de l'axe longitudinal,
l'effecteur terminal comprenant en outre :
une liaison mécanique liant la pluralité d'éléments de boucle au niveau du sommet distal, la liaison mécanique comprenant au moins l'une parmi :
une forme rectangulaire ou ovoïde comprenant une ouverture à travers laquelle la pluralité d'éléments de boucle s'étendent et un côté comprenant une jointure ;
une forme rectangulaire ou ovoïde comprenant une ouverture à travers laquelle la pluralité d'éléments de boucle s'étendent et quatre côtés contigus ;
une forme rectangulaire ou ovoïde comprenant trois ouvertures comprenant chacune un élément de boucle respectif de la pluralité d'éléments de boucle s'étendant à travers celles-ci ;
une forme cylindrique comprenant une pluralité de voies de passage à travers celle-ci, la pluralité de voies de passage comprenant chacune un élément de boucle respectif de la pluralité d'éléments de boucle s'étendant à travers celles-ci ; et
un anneau effilé comprenant une ouverture annulaire à travers laquelle la pluralité d'éléments de boucle s'étendent et une hauteur effilée s'étendant à travers un diamètre de l'ouverture annulaire ;
une pluralité d'électrodes attachées à la pluralité d'éléments de boucle, chaque électrode de la pluralité d'électrodes comprenant une surface **caractérisée par** un paramètre de rugosité Ra représentant une déviation moyenne arithmétique d'un profil de la surface, où Ra mesure d'environ 0,3 micromètre à environ 0,4 micromètre ; et
au moins un fil de traction s'étendant à travers une tige allongée et fixé à une partie distale de la tige allongée de sorte que lorsque le fil de traction est rétracté en direction d'une partie proximale par rapport à la tige allongée, la partie distale de la tige allongée et l'effecteur terminal sont pliés selon un angle par rapport à l'axe longitudinal.

7. Effecteur terminal selon la revendication 1, l'élément de châssis secondaire étant conçu pour coulisser dans au moins une première dimension par rapport à l'élément de châssis primaire ; et chaque élément de boucle comprenant en outre :
un manchon disposé autour de l'au moins un des éléments de châssis primaire et secondaire.

8. Effecteur terminal selon la revendication 7,
la première dimension étant longitudinale ;
l'élément de châssis primaire comprenant une bande de fil métallique et s'étendant longitudinalement le long d'au moins une première partie d'une longueur de l'ensemble de boucles ; et
l'élément de châssis secondaire comprenant une base métallique et s'étendant longitudinalement le long d'au moins une seconde partie de la longueur de l'ensemble de boucles, la première partie de la longueur de l'ensemble de boucles chevauchant au moins partiellement la seconde partie de la longueur de l'ensemble de boucles au niveau d'une section de chevauchement.

9. Effecteur terminal selon la revendication 8,
l'élément de châssis primaire étant formé par une bande de fil plat en nitinol ; et
l'élément de châssis secondaire étant formé d'une base en nitinol ayant une épaisseur plus grande que la bande de fil plat en nitinol.

10. Effecteur terminal selon la revendication 9,
(a) la bande de fil plat en nitinol formant l'élément de châssis primaire ayant une épaisseur d'environ 0,002 pouce ; et
la base en nitinol formant l'élément de châssis secondaire ayant une épaisseur d'environ 0,005 pouce ; ou
(b) la bande de fil plat en nitinol formant l'élément de châssis primaire ayant une épaisseur comprise d'environ 25 % à environ 30 % d'une épaisseur de l'ensemble de boucles dans la section de chevauchement ; et
la base en nitinol formant l'élément de châssis secondaire ayant une épaisseur comprise d'environ 70 % à environ 75 % de l'épaisseur de l'ensemble de boucles dans la section de chevauchement.

11. Effecteur terminal selon la revendication 7, comprenant en outre :
une tige allongée comprenant une partie proximale et une partie distale, la tige allongée étant conçue pour être manipulée au niveau de la partie proximale pour positionner la partie distale dans un cœur d'un patient, la tige allongée s'étendant le long de l'axe longitudinal, et
l'élément de châssis primaire étant fixé à l'élément de châssis secondaire seulement par l'intermédiaire d'un manchonnage thermorétractable.

12. Ensemble destiné à un cathéter de cartographie comprenant :
un élément tubulaire s'étendant le long d'un axe longitudinal ;
l'effecteur terminal selon la revendication 1, chaque châssis de support étant attaché à une partie distale de l'élément tubulaire, chaque châssis de support s'étendant à travers l'élément de boucle respectif pour définir deux éléments d'ossature centrale s'étendant à partir de l'élément tubulaire et reliés à un élément arqué ; et
chaque élément de boucle comprenant en outre : une pluralité d'électrodes disposées sur le châssis de support respectif ;
au moins une partie de chacun des éléments d'ossature centrale comprenant les éléments de châssis primaire et secondaire s'étendant longitudinalement, l'élément de châssis primaire étant conçu pour coulisser au moins dans une première dimension par rapport à l'élément de châssis secondaire.

13. Ensemble selon la revendication 12,
la première dimension étant longitudinale ;
un premier élément de boucle de la pluralité d'éléments de boucle étant disposé en réseau sur un premier plan ;
un deuxième élément de boucle de la pluralité d'éléments de boucle étant disposé en réseau sur un deuxième plan qui croise le premier plan ; et
un troisième élément de boucle de la pluralité d'éléments de boucle étant disposé en réseau sur un troisième plan qui croise le premier plan et le deuxième plan de telle sorte qu'une majorité d'une longueur de chacun des trois éléments de boucle est non coplanaire avec la majorité de la longueur d'au moins l'un des trois autres éléments de boucle dans une configuration sans contrainte.

14. Ensemble selon la revendication 12,
au moins une partie de chacun des éléments d'ossature centrale de chacun des châssis de support comprenant les éléments de châssis primaire et secondaire s'étendant longitudinalement, l'élément de châssis primaire de chacun des châssis de support étant conçu pour coulisser longitudinalement par rapport à l'élément de châssis secondaire du châssis de support respectif ;
l'élément de châssis primaire de chacun des châssis de support comprenant une bande de fil formée d'un premier matériau ; et
l'élément de châssis secondaire de chacun des châssis de support comprenant une base formée du premier matériau et ayant une coupe transversale plus épaisse qu'une coupe transversale respective de la bande de fil.

15. Ensemble selon la revendication 14,
le premier matériau comprenant du nitinol ;
la bande de fil en nitinol formant l'élément de châssis primaire de chaque châssis de support ayant une épaisseur comprise d'environ 25 % à environ 30 % d'une épaisseur du châssis de support respectif ; et
la base en nitinol formant l'élément de châssis secondaire de chaque châssis de support ayant une épaisseur comprise d'environ 70 % à environ 75 % de l'épaisseur du châssis de support respectif, facultativement l'épaisseur de la bande de fil en nitinol mesurant environ 0,002 pouce ; et
l'épaisseur de la base en nitinol mesurant environ 0,005 pouce.

16. Ensemble selon la revendication 14,
l'élément de châssis primaire de chacun des châssis de support étant confiné aux éléments d'ossature centrale du châssis de support respectif ; et
l'élément de châssis secondaire de chacun des châssis de support étant formé d'une couche de base en nitinol d'un seul tenant s'étendant sur l'ensemble des éléments d'ossature centrale et de l'élément arqué du châssis de support respectif.

17. Ensemble selon la revendication 14,
l'élément de châssis primaire de chacun des châssis de support s'étendant sur une longueur entière des éléments d'ossature centrale du châssis de support respectif dans une configuration sans contrainte ; et
l'élément de châssis primaire de chacun des châssis de support s'étendant uniquement sur une partie de la longueur entière des éléments d'ossature centrale du châssis de support respectif lorsque la pluralité d'éléments de boucle sont fléchis selon un angle par rapport à l'axe longitudinal.

18. Ensemble selon la revendication 14,
l'élément de châssis primaire de chacun des châssis de support étant fixé à l'élément de châssis secondaire du châssis de support respectif seulement par l'intermédiaire d'un manchonnage thermorétractable.
